Europäisches Patentamt

**European Patent Office**

Office européen des brevets

(19)

(11) Numéro de publication: **0 196 258 B1**

(12) **FASCICULE DE BREVET EUROPEEN**

(45) Date de publication du fascicule du brevet:
18.10.89

(51) Int. Cl.⁴: **A 61 F 2/36**

(21) Numéro de dépôt: **86400616.8**

(22) Date de dépôt: **24.03.86**

---

(54) Composant fémoral de prothèse de hanche, non cimenté.

---

(30) Priorité: **27.03.85 FR 8504548**

(43) Date de publication de la demande:
**01.10.86 Bulletin 86/40**

(45) Mention de la délivrance du brevet:
**18.10.89 Bulletin 89/42**

(84) Etats contractants désignés:
**AT BE CH DE GB IT LI LU NL SE**

(56) Documents cités:
**EP-A- 0 041 591**
**EP-A- 0 050 533**
**EP-A- 0 085 147**
**EP-A- 0 128 036**
**EP-A- 0 131 178**
**EP-A- 0 149 527**
**WO-A-84/03037**
**DE-A- 2 839 093**
**FR-A- 1 278 359**
**US-A- 4 163 292**

(73) Titulaire: **Epinette, Jean-Alain, 27 rue Lamandin, F-62700 Bruay-en-Artois (FR)**

(72) Inventeur: **Epinette, Jean-Alain, 27 rue Lamandin, F-62700 Bruay-en-Artois (FR)**

(74) Mandataire: **CABINET BONNET-THIRION, 95 Boulevard Beaumarchais, F-75003 Paris (FR)**

ACTORUM AG

## Description

L' invention a trait à un composant fémoral de prothèse de hanche destiné à être implanté sans ciment, et comportant une tige adaptée à être enfoncée dans la cavité médullaire du fémur après ablation de la tête par section du col, s'effilant jusqu'à une extrémité inférieure depuis une extrémité supérieure d'où fait saillie un col portant une tête sphérique rapportée, adaptée à former rotule avec un composant cotyloïdien en sorte de reproduire l'articulation naturelle originaire.

Il est bien connu que, dans leur structure, les prothèses de hanche constituent articulation à rotule, avec un composant femelle reconstituant la cavité cotyloïde dans l'os iliaque, et un composant mâle portant une tête sphérique emmanchée à l'extrémité d'un col, celui-ci se raccordant à une tige ancrée dans la cavité médullaire du fémur. L'invention porte sur ce dernier composant, ou composant fémoral, et ne se référera au composant cotyloïdien qu'à propos de sa complémentarité avec le composant fémoral.

On comprendra que, la prothèse devant se substituer à l'articulation naturelle de la tête du fémur dans la cavité cotyloïde de l'os iliaque, dans des conditions aussi proches que possible de l'articulation naturelle saine, les éléments des composants de prothèse qui viennent prendre la place des éléments osseux de l'articulation seront désignés par les mêmes noms que ces éléments osseux, leurs orientations et dimensions seront, sauf indications contraires, semblables aux éléments d'articulation naturelle correspondants, et les références à leurs positions spatiales seront données par rapport à leur position implantée.

Plus particulièrement on considérera que, par rapport à l'axe général du fémur, considéré comme approximativement droit et vertical, la tête est déportée dans un plan frontal du côté interne à l'extrémité du col, orienté dans ce plan frontal en formant un angle d'environ 135° avec l'axe du fémur, et que l'axe du col se raccorde avec l'axe du fémur par une incurvation de l'axe du canal médullaire dans la région du trochanter, cet axe formant sensiblement la fibre neutre du fémur considéré comme élément porteur Il existe deux grands types de composants fémoraux de prothèse de hanche, les composants qui sont scellés avec un ciment organique polymérisé dans la cavité médullaire, et les composants qui sont bloqués à force dans cette cavité, la croissance de l'os spongieux au contact de la surface de la tige, convenablement conformée avec de nombreuses cavités et saillies, amenant un ancrage par interpénétration de l'os et de la surface de la tige.

Les composants qui sont scellés à l'aide d'un ciment organique présentent un certain nombre d'inconvénients ; lors de la pose de la prothèse, il est nécessaire d'attendre, après le scellement de la tige, que le ciment ait acquis une résistance suffisante par polymérisation pour poursuivre l'intervention. La prise du ciment par polymérisation dégage de la chaleur, et induit un certain foisonnement du ciment, ce qui provoque parfois des douleurs pénibles. La présence du ciment ne favorise pas la croissance de l'os spongieux, et il peut arriver qu'au cours du temps la tige scellée prenne du jeu, ce qui impose une nouvelle intervention. Enfin, lors d'une nouvelle pose de prothèse, l'élimination du ciment antérieur peut être difficile.

On a préconisé l'utilisation de composants fémoraux tenus bloqués dans la cavité médullaire. La tige présente un rayon de courbure différent de celui de l'axe de la cavité médullaire au raccordement avec le col du fémur. Quand cette tige a été enfoncée dans la cavité médullaire, elle porte sur les parois de cette cavité en trois zones ; aux deux extrémités, pointe de la tige et racine du col elle porte par sa génératrice interne la plus concave, et dans la région du trochanter elle porte par sa génératrice dorsale, en une zone intermédiaire entre les extrémités, la génératrice dorsale étant opposée à la génératrice interne dans le plan frontal. Comme il a déjà été signalé, la face extérieure de la tige présente un état de surface, parfois dit madréporique, avec des cavités et des saillies, pour favoriser la pénétration et l'accrochage de l'os spongieux en croissance. D'autres composants fémoraux sont massifs et se bloquent sur toute leur longueur. Leur tige est généralement plus longue que celle des précédents.

L'étude du comportement de tels composants fémoraux après mise en place, a fait apparaître que le blocage ainsi obtenu n'était pas sans inconvénients. L'effort de la tige est générateur de douleur pour le sujet. Par ailleurs il est apparu que si le blocage en rotation de la tige était indispensable, le bon fonctionnement de la prothèse impliquait une souplesse longitudinale, avec un jeu de l'ordre du millimètre. En effet si le métal, généralement titane pour des raisons de poids, compatibilité biologique, inertie chimique, usinabilité, présente un module d'élasticité le moins éloigné de celui de l'os cortical, il ne reproduit pas la souplesse et l'amortissement de l'os naturel dans la région du trochanter et du col. Or, avec le blocage en trois points, la souplesse s'obtient par des déplacements longitudinaux de la tige qui provoquent des jeux aux zones d'appui, ces jeux n'assurant pas suffisamment le blocage en rotation. Les composants fémoraux massifs ne présentent pas la souplesse requise.

On a proposé également (EP-A-0 131 178) un composant fémoral avec une broche, portant la tête d'articulation, qui se rétrécit du haut vers le bas, et un ensemble de pièces en coin, qui sont guidées librement dans des rainures pratiquées dans la broche. Le frottement entre pièces en coin et rainures de broche est plus faible que celui qui s'établit entre l'os et les surfaces externes des pièces en coin, qui sont adaptées à être adhérentes. La charge de la broche sollicite en écartement les pièces en coin; si la cavité médullaire s'élargit, l'enfoncement de la broche évite une prise de jeu entre l'os et le composant. Le document précise que l'angle de coincement de la broche est plus faible que l'angle naturel de frottement entre broche et pièces en coin, pour éviter un jeu de va-et- vient entre ces pièces. Ainsi la broche est bloquée contre tout déplacement relatif par rapport à l'os, quelle qu'en soit la direction, à l'exception de l'enfoncement de la broche pour la reprise de jeu en cas d'élargissement de la cavité médullaire.

Cette disposition empêche totalement un jeu en rotation du composant fémoral par rapport à l'os, mais au prix de la suppression de toute souplesse longitudinale. On retrouve les inconvénients des composants massifs.

Pour pallier ces inconvénients, l'invention propose un composant fémoral de prothèse de hanche, destiné à être implanté sans ciment et comportant une tige adaptée à être enfoncée dans la cavité médullaire du fémur après ablation de la tête par section du col, s'effilant jusqu'à une extrémité inférieure depuis une extrémité supérieure d'où fait saillie un col portant une tête sphérique rapportée, adaptée à former rotule avec un composant cotyloïdien, en sorte de reproduire l'articulation naturelle originaire, et présentant une incurvation correspondant au raccordement naturel, dans un plan frontal défini par référence anatomique, de l'axe du col à l'axe de la diaphyse en passant par le trochanter, la tige étant composée d'une âme et de cales et présentant, au moins dans la région du trochanter un état de surface apte à une liaison avec de l'os spongieux en croissance, composant caractérisé en ce que ladite âme est limitée, parallèlement au plan frontal, par deux faces frontales antérieure et postérieure généralement planes s'étendant au moins dans la région du trochanter, et en ce que deux cales sont adaptées à s'accoler à l'âme suivant les faces frontales antérieure et postérieure par des faces d'appui complémentaires de ces faces de l'âme en sorte de laisser libre un déplacement relatif au moins limité de l'âme par rapport aux cales, en seule translation suivant le plan frontal, les cales présentant en outre des faces convexes adaptées à épouser la cavité médullaire et présentant ledit état de surface apte à la liaison avec de l'os spongieux.

Alors que les cales peuvent se fixer dans une position définitive, l'âme peut garder un jeu de déplacement longitudinal appréciable en coulissant entre les faces d'appui des cales, qui empêchent toute rotation. La tige dans son ensemble, et plus précisément l'âme, n'est pas bloquée par flexion de l'arc, ce qui supprime une des causes de souffrance pour le sujet. Par ailleurs, l'âme repose, dans le sens longitudinal, sur de l'os, par la majorité de sa surface extérieure aux faces frontales planes, ce qui permet de retrouver une souplesse et un amortissement comparables à ceux de l'os originaire.

Le document EP-A-O 149 157, publié postérieurement à la date de priorité dont bénéficie le présent titre, ne fait partie de l'état de la technique que sous l'aspect de la nouveauté selon l'article 54(3) CBE. Il décrit une prothèse susceptible de jeu axial par rapport au fémur quelle équipe. Ce jeu axial est obtenu en donnant à la prothèse une forme de lame plane avec des arêtes interne et externe, et en garnissant la cavité médullaire, en partie haute, de deux cales qui chevauchent les arêtes, en sorte que la charge de l'articulation force les cales contre le cortex. En variante, les cales ont la même épaisseur que les arêtes, et deux cales planes supplémentaires antérieure et postérieure assurent la stabilité de la prothèse.

En disposition préférée l'âme du composant selon l'invention comporte une lame dorsale dans le plan frontal, du côté convexe de l'incurvation, cette lame étant apte à s'enfoncer dans le trochanter. Cette lame assure un blocage supplémentaire en pivotement, et une orientation définie de l'âme dans la cavité.

De préférence les faces d'appui des cales sont garnies d'une couche de polymère biologiquement compatible. Cette disposition réduit le frottement entre cales et âme.

De préférence également les faces frontales de l'âme et les faces d'appui des cales présentent des moyens de guidage complémentaires, moyens de guidage aptes à diriger les cales, par rapport à l'âme, dans la cavité médullaire. L'âme étant mise en place en premier lieu, l'insertion des cales et leur prise de position sont facilitées par les moyens de guidage.

Selon une disposition avantageuse, chaque cale comporte une cavité ouverte à la partie supérieure, et fermée suivant la face convexe par une paroi ajourée. Des greffons d'os spongieux peuvent être insérés dans la cavité pour se souder, par croissance à l'os spongieux qui garnit la cavité médullaire, à travers les ajours de la paroi convexe de cale. Les cales sont ainsi parfaitement incorporées à la paroi de la cavité médullaire.

Les caractéristiques et avantages de l'invention ressortiront d'ailleurs de la description qui va suivre, à titre d'exemple, en référence aux dessins annexés dans lesquels:

la figure 1 représente schématiquement une prothèse de hanche avec un composant fémoral selon l'invention;

la figure 2 est une perspective éclatée du composant fémoral de la figure 1, composé d'une âme et deux cales;

la figure 3 est une vue de la partie supérieure de l'âme du composant fémoral de l'invention;

la figure 4 est une coupe suivant le plan IV–IV de la figure 3;

les figures 5A et 5B représentent en coupe, en correspondance de la coupe de la figure 3, des cales antérieure et postérieure du composant fémoral.

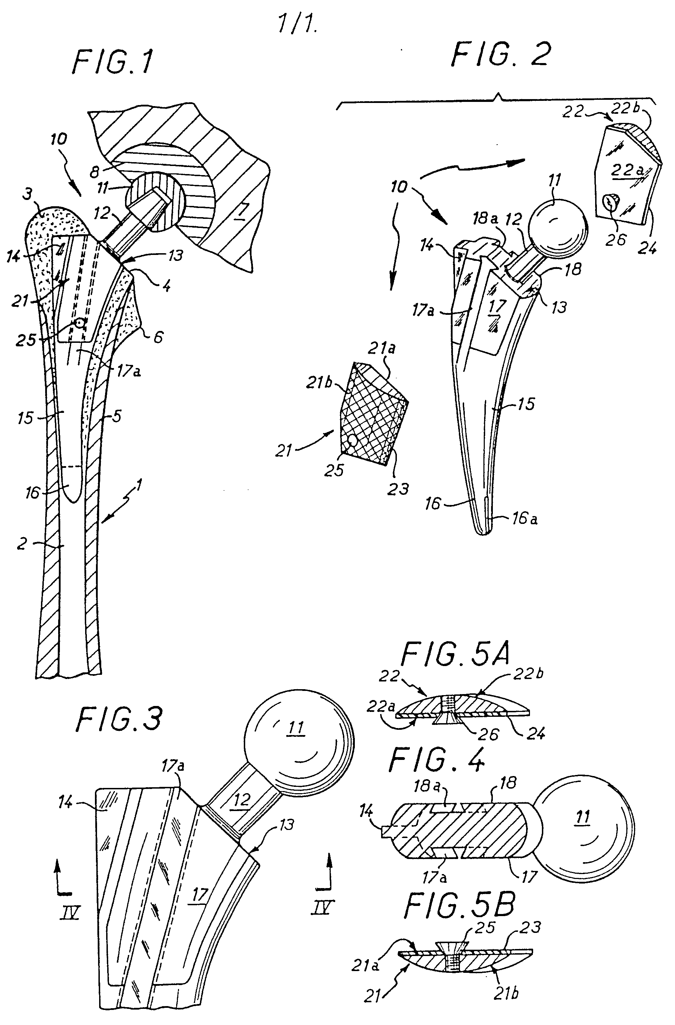

Comme représenté figure 1, une prothèse de hanche comporte, implanté dans le fémur 1, un composant fémoral 10 dans son ensemble avec une tige 15 enfoncée dans le canal médullaire 2 du fémur 1. Saillant de l'extrémité supérieure 13 de la tige 15, un col cylindrique 12 porte une tête 11, col 12 et tête 11 étant dimensionnés pour se substituer aux col et tête originaires. La tête 11, sphérique, rotule dans un composant cotyloïdien 8, fixé dans l'os iliaque 7 (généralement par vissage).

Le fémur 1 comporte, à son extrémité supérieure, des masses d'os spongieux, comprenant: le grand trochanter 3, en saillie divergeant du col, dans un plan frontal qui correspond au plan de la figure ; le petit trochanter 6 qui fait saillie dans ce plan frontal à la face interne du fémur (c'est-dire la face la plus proche du fémur de l'autre cuisse). Le fût cortical du fémur 1 s'évase et se termine en lame, la cavité médullaire 2 étant également évasée et garnie vers son extrémité supérieure d'os spongieux, notamment le calcar 4 vers le bord interne de la cavité médullaire, à proximité du petit trochanter 6.

Selon la forme de réalisation choisie, plus apparentée sur la figure 2, le composant fémoral 10 comprend une âme 15 en titane forgé de nuance TA6V, qui constituée la partie la plus importante de la tige du composant fémoral 10. Cette âme s'effilée depuis sa face supérieure 13 jusqu'à son extrémité inférieure 16 et présente deux faces frontales 17 et 18, parallèles au plan frontal qui contient l'axe de la tige. Si la figure 1 représente une articulation droite du sujet vu de face, les faces 17 et 18 seront respectivement antérieure et postérieure. On notera que, sur la figure 2, la face postérieure 18 est cachée.

L'extrémité inférieure 16 de l'âme présente une fente 16a suivant le plan frontal, pour conférer de l'élasticité suivant une direction sagittale, perpendiculaire au plan frontal ; cette disposition autorise des déplacements longitudinaux de l'âme 15, dans la partie resserrée de la cavité médullaire 2, les becs de l'âme, de part et d'autre de la fente 16a, formant ressort en appui sur la paroi.

Des cales 21 et 22 également en titane forgé viennent s'accoler, par des faces planes d'appui 21a et 22a sur les faces planes frontales respectivement antérieure 17 et postérieure 18 de l'âme 15. Les cales 21 et 22 présentent en outre des faces externes convexes 21b et 22b qui, en position d'accolement, viennent compléter une forme complémentaire de la forme en entonnoir de la cavité médullaire.

Comme pratiquement toutes les tiges de composants fémoraux, l'âme 15 est incurvée pour raccorder l'axe du col 12 à l'axe de la cavité médullaire 2, en reproduisant la disposition naturelle de l'articulation. L'incurvation est essentiellement dans le plan frontal, et est concave pour la génératrice interne.

Par ailleurs, pour améliorer le blocage en rotation de la tige, la génératrice externe de la tige 15, ou génératrice dorsale, est munie d'une lame dorsale 14, qui pénètre à force dans le grand trochanter lors de l'insertion de l'âme 15 dans le fémur 1.

Comme on peut le voir mieux sur les figures 3 et 4, dans les faces frontales 17 et 18 de l'âme 15 sont usinées deux rainures en queue d'aronde 17a et 18a droites et débouchant dans la face supérieure 13 de l'âme 15 à hauteur du col 12. Ces rainures en queue d'aronde sont destinées à guider les cales 21 et 22 lors de leur mise en place.

Comme on le voit sur les figures 5A et 5B, les cales 21 et 22 sont garnies, sur leurs faces d'appui 21a et 22a de couches 23 et 24 respectivement en polyéthylène haute densité. En outre, vers leurs extrémités inférieures, les cales comportent des plots 25 et 26 respectivement à tête tronconique et tige filetée, exécutés en polyéthylène haute densité, le profil des têtes tronconiques des plots 25 et 26 venant s'inscrire dans les rainures en queue d'aronde 17a 18a pour guider les extrémités inférieures des cales 21 et 22.

Les faces externes 21b et 22b des cales 21 et 22 ont été rendues rugueuses, avec des pores de 0,2 à 0,5 mm, soit par microbillage, soit par un recouvrement de poudre de titane fritté, soit par pulvérisation à la torche à plasma.

La pose d'une prothèse de hanche présente deux aspects, un aspect d'intervention chirurgicale qui échappe de soi au cadre de l'invention, et un aspect de procédé mécanique qui a déterminé la structure de l'objet de l'invention. C'est dans cette optique de procédé mécanique que la description de la mise en place de la prothèse sera donnée. On comprendra toutefois que le processus de mise en place dépend des réactions organiques.

La mise en place du composant cotyloïdien ne sera évoquée qu'en passant, étant donné qu'elle est en dehors du cadre de l'invention, mais que la mise en place du composant fémoral en est nécessairement dépendante.

Pour poser le composant fémoral, on coupe le col au ras du calcar 4 sous un angle d'environ 45° par rapport à la direction générale du fémur, et on dégage le canal médullaire en ménageant une cavité à la forme de la tige de composant fémoral 10, dont la taille a été déterminée par des examens radiographiques antérieurement, en prévoyant un serrage.

L'âme 15 est alors mise en place à force, par «impaction» selon l'expression de technique chirurgicale jusqu'à ce que sa face d'extrémité supérieure 13 vienne sensiblement au ras de la section du col de fémur, cette mise en place s'exécutant dans un plan frontal.

On choisit alors, dans des jeux de cales antérieures 21 et postérieures 22 de convexité croissante, les cales qui correspondent au mieux à la forme de la cavité médullaire. Les plots 25 et 26 de ces cales 21 et 22 sont engagés respectivement dans l'entrée des rainures 17a et 18a de l'âme 15, et les cales sont amenées à leur place définitive par «impaction».

On notera que, lors de la mise en place des cales 21 et 22, l'orientation de ces cales par rapport à l'âme 15 varie, de sorte que les cales peuvent suivre dans leur ensemble une trajectoire courbe, correspondant à l'incurvation de l'âme, alors que les rainures de guidage sont rectilignes.

Comme on l'a précisé plus haut, les surfaces convexes 21b et 22b des cales 21 et 22 sont rugueuses et madréporiques pour faciliter la liaison avec l'os spongieux en croissance. Dans une autre réalisation, les cales sont creusées de rainures débouchant dans la tranche supérieure, et les surfaces convexes 21b et 22b sont constituées de toiles métalliques en fil de titane, soudées à leur périphérie sur le corps de cale. Il est alors possible d'insérer dans les cales des greffons d'os spongieux, qui croîtront et viendront se lier aux parois de la cavité médullaire à travers la toile métallique.

L'immobilisation des cales dans la partie supérieure du fémur est ainsi assurée pour de longues périodes.

On comprendra que l'âme, qui porte sur les garnitures en polyéthylène des faces d'appui des cales, est susceptible de se déplacer longitudinalement sur un trajet millimétrique, ce qui confère à la prothèse une souplesse et un amortissement comparable à celle de l'articulation saine.

Dans une variante de l'invention, on a supprimé la lame dorsale 14, et les garnitures en polyéthylène 23 et 24 des cales latérales 21 et 22. Les faces frontales 17 et 18 de l'âme 15 ne sont plus strictement parallèles, mais se fondent dans l'effilement général de cette âme. Toutefois les fonds de rainures 17a et 18a sont strictement parallèles, et les plots 25 et 26 sont en titane et présentent une longueur telle que le contact entre cales 21 et 22, et âme 15 se fait essentiellement par l'appui des tranches terminales des plots 25 et 26 sur les fonds de rainures 17a et 18a, de sorte que les possibilités de déplacement millimétrique de l'âme 15 par rapport aux cales 21 et 22 en réponse à des chargées verticales variables de la prothèse soient maintenues.

La surface de l'âme 15, ainsi que les faces d'appui des cales 21 et 22 sont nitrurées, pour améliorer la compatibilité physiologique de la prothèse.

Bien entendu l'invention n'est pas limitée à l'exemple décrit en détail, mais en embrasse les variantes dans le cadre de la portée des revendications.

## Revendications

1. Composant fémoral de prothèse de hanche, destiné à être implanté sans ciment et comportant une tige adaptée à être enfoncée dans la cavité médullaire du fémur après ablation de la tête par section du col, s'effilant jusqu'à une extrémité inférieure (16) depuis une extrémité supérieure (13) d'où fait saillie un col (12) portant une tête sphérique (11) rapportée, adaptée à former rotule avec un composant cotyloïdien (8), en sorte de reproduire l'articulation naturelle originaire, et présentant une incurvation correspondant au raccordement naturel, dans un plan frontal défini par référence anatomique, de l'axe du col à l'axe de la diaphyse (1) en passant par le trochanter (3), la tige étant composée d'une âme (15) et de cales (21, 22) et présentant, au moins dans la région du trochanter (3) un état de surface apte à une liaison avec de l'os spongieux en croissance, composant caractérisé en ce que ladite âme (15) est limitée, parallèlement au plan frontal, par deux faces frontales antérieure (17) et postérieure (18) généralement planes s'étendant au moins dans la région du trochanter (3), et en ce que deux cales (21, 22) sont adaptées à s'accoler à l'âme (15) suivant les faces frontales antérieure (17) et postérieure (18) par des faces d'appui (21a, 22a) complémentaires de ces faces de l'âme en sorte de laisser libre un déplacement relatif au moins limité de l'âme (15) par rapport aux cales (21, 22), en seule translation suivant le plan frontal, les cales présentant en outre des faces convexes (21b,

22b) adaptées à épouser la cavité médullaire et présentant ledit état de surface apte à la liaison avec de l'os spongieux.

2. Composant fémoral selon la revendication 1, caractérisé en ce que l'âme (15) comporte une lame dorsale (14) dans le plan frontal, du côté convexe de l'incurvation, apte à s'enfoncer dans le trochanter (3).

3. Composant fémoral selon l'une des revendications 1 et 2, caractérisé en ce que l'âme (15) présente à son extrémité inférieure (16), une fente (16a) étendue suivant le plan frontal.

4. Composant fémoral selon l'une quelconque des revendications 1 à 3, caractérisé en ce que les faces d'appui (21a, 22a) des cales sont garnies d'une couche (23, 24) d'un polymère biologiquement compatible.

5. Composant fémoral selon l'une quelconque des revendications 1 a 4, caractérisé en ce que les faces frontales (17, 18) de l'âme et les faces d'appui (21a, 22a) des cales présentent des moyens de guidage ( 17a, 18a, 25, 26) complémentaires, ces moyens de guidage étant aptes à diriger les cales (21, 22) par rapport à l'âme (15) dans la cavité médullaire.

6. Composant fémoral selon la revendication 5, caractérisé en ce que les moyens de guidage sont constitués, pour l'âme (15), par une rainure en queue d'aronde (17a, 18a) dans chaque face frontale ( 17, 18) , et pour chaque cale (21, 22), d'un plot (25, 26) à tête conique en saillie sur la face d'appui.

7. Composant fémoral selon la revendication 6, caractérisé en ce que le plot (25, 26) à tête conique est situé vers la partie inférieure de la cale (21, 22).

8. Composant fémoral selon l'une des revendications 6 et 7, caractérisé en ce que le plot à tête conique (25, 26) est constitué d'un polymère biologiquement compatible, avec une tige vissée dans la face d'appui de la cale (21, 22).

9. Composant fémoral selon l'une quelconque des revendications 1 à 8, caractérisé en ce que chaque cale (21, 22) comporte une cavité ouverte à la partie supérieure de la cale et fermée suivant la face convexe (21b, 22b) par une paroi ajourée.

10. Composant fémoral selon la revendication 7, caractérisé en ce que, les fonds des rainures (17a, 18a) usinées dans les faces antérieure (17) et postérieure (18) de l'âme (15) étant strictement parallèles au plan frontal, les plots coniques (25, 26) en saillie des cales (21, 22) portent par une tranche terminale sur les fonds des rainures (17a, 18a) correspondants, en sorte de maintenir le déplacement de l'âme (15) par rapport aux cales (21, 22) en seule translation suivant le plan frontal, alors que les faces (17, 18) se fondent, en partie inférieure, dans l'effilement général de l'âme.

## Patentansprüche

1. Femurteil einer Hüftgelenkprothese zur zementfreien Implantation mit einem Schaft zum Einpressen in die Knochenmarkhöhle des Femurs nach Abtragen von dessen Kopf durch Abschneiden des Halses, wobei sich der Schaft bis zu einem unteren Ende (16) verjüngt, ausgehend von einem oberen Ende (13), von wo ein Hals (12) vorspringt, der einen angesetzten Kugelkopf (11) trägt, der so ausgebildet ist, dass er mit einem Gelenkpfannenteil (8) die Gelenkkugel bildet, um das ursprüngliche natürliche Gelenk nachzubilden, und eine der natürlichen Zusammenfügung entsprechende Einwölbung in einer durch anatomischen Bezug definierten Frontebene von der Achse des Halses zur Achse der Diaphysis (1) unter Durchgang durch den Rollhügel (3) aufweist, wobei der Schaft zusammengesetzt ist aus einer Seele (15) und Einsätzen (21, 22) und mindestens im Bereich des Rollhügels (3) einen Oberflächenzustand aufweist, der für eine Verbindung mit dem wachsenden spongiösen Knochen geeignet ist, wobei der Femurteil dadurch gekennzeichnet ist, dass die Seele (15) parallel zur Frontebene durch zwei im ganzen ebene Frontflächen, eine vordere (17) und hintere (18) Frontfläche begrenzt ist, die sich mindestens im Bereich des Rollhügels (3) erstrecken, und dass zwei Einsätze (21, 22) so ausgebildet sind, dass sie sich an die Seele (15) gemäss der vorderen Frontseite (17) und hinteren Frontseite (18) mit Auflageflächen (21a, 22a), die zu diesen Flächen der Seele komplementär sind, so anlegen, dass eine zumindest begrenzte Relativbewegung der Seele (15) bezüglich der Einsätze (21, 22) nur in Verschiebung gemäss der Frontebene möglich bleibt, wobei die Einsätze ausserdem konvexe Flächen (21b, 22b) aufweisen, die so ausgebildet sind, dass sie der Knochenmarkhöhle genau entsprechen, und die den zur Verbindung mit dem spongiösen Knochen geeigneten Oberflächenzustand aufweisen.

2. Femurteil nach Anspruch 1, dadurch gekennzeichnet, dass die Seele (15) in der Frontebene einen dorsalen Sporn (14) auf der konvexen Seite der Biegung aufweist, der zum Einpressen in den Rollhügel (3) geeignet ist.

3. Femurteil nach einem der Ansprüche 1 und 2, dadurch gekennzeichnet, dass die Seele (15) an ihrem unteren Ende (16) einen gemäss der Frontebene verlaufenden Schlitz (16a) aufweist.

4. Femurteil nach einem der Ansprüche 1 bis 3, dadurch gekennzeichnet, dass die Auflageflächen (21a, 22a) der Einsätze mit einer Schicht (23, 24) aus einem bioligisch veträglichen Polymer versehen sind.

5. Femurteil nach einem der Ansprüche 1 bis 4, dadurch gekennzeichnet, dass die Frontflächen (17, 18) der Seele und die Auflageflächen (21a, 22a) der Einsätze komplementäre Führungsvorrichtungen (17a, 18a; 25, 26) aufweisen, die geeignet sind, die Einsätze (21, 22) bezüglich der Seele (15) in der Knochenmarkhöhle zu führen.

6. Femurteil nach Anspruch 5, dadurch gekennzeichnet, dass die Führungsvorrichtungen hinsichtlich der Seele (15) aus einer Schwalbenschwanznut (17a, 18a) in jeder Frontfläche (17, 18) und hinsichtlich jedes Einsatzes (21, 22) aus einem von der Auflagefläche vorspringenden Klotz (25, 26) mit kegelförmigem Kopf bestehen.

7. Femurteil nach Anspruch 6, dadurch gekennzeichnet, dass der Klotz (25, 26) mit Kegelkopf im unteren Teil des Einsatzes (21, 22) angeordnet ist.

8. Femurteil nach einem der Ansprüche 6 und 7, dadurch gekennzeichnet, dass der Klotz mit Kegelkopf (25, 26) aus einem biologisch verträglichen Polymer besteht mit einem Stift, der in die Auflagefläche des Einsatzes (21, 22) eingeschraubt ist.

9. Femurteil nach einem der Ansprüche 1 bis 8, dadurch gekennzeichnet, dass jeder Einsatz (21, 22) am oberen Teil des Einsatzes eine offene Höhlung aufweist, die gemäss der konvexen Seite (21b, 22b) durch eine durchbrochene Wand verschlossen ist.

10. Femurteil nach Anspruch 7, dadurch gekennzeichnet, dass die Böden der in der vorderen Fläche (17) und hinteren Fläche (18) der Seele (15) eingeschnittenen Nuten (17a, 18a) genau parallel zur Frontebene sind und die von den Einsätzen (21, 22) vorspringenden konischen Klötze (25, 26) mit einer Endfläche auf den entsprechenden Böden der Nuten (17a, 18a) aufliegen, so dass die Bewegung der Seele (15) bezüglich der Einsätze (21, 22) nur in Verschiebung gemäss der Frontebene ermöglicht wird, während die Seiten (17, 18) im unteren Teil in die allgemeine Verjüngung der Seele übergehen.

## Claims

1. A femoral component of a hip joint replacement for cement-free Implantation and comprising a stem engageable in the medullary cavity of the femur after removal of the femoral head by resectioning of the neck, the stem narrowing to a bottom end (16) from a top end (13) from which a neck (12) carrying a fitted spherical head (11) projects, the head being adapted to co-operate with an acetabular component (8) to form a swivel joint so as to reproduce the original natural joint, the stem having a curvature corresponding to the natural connection, in a frontal plane defined by anatomical reference, between the neck axis and the shaft (1) axis by way of the trochanter (3), the stem comprising a core (15) and shims (21, 22) and having at least in the trochanter region a surface texture suitable for connection to the spongey growth tissue of the bone, characterised in that the core (15) is bounded parallel to the frontal plane by two frontal surfaces, namely an anterior surface (17) and a posterior surface (18), which are substantially plane and which extend at least into the region of the trochanter (3), and two shims (21, 22) are adapted to engage the core (15) on its anterior and posterior frontal surfaces (17, 18) by way of bearing surfaces (21a, 22a respectively) which are complementary to the said anterior and posterior faces so as to leave the core (15) with at least relative provision for movement relatively to the shims (21, 22) solely in translation in the frontal plane, the shims also having convex surfaces (2lb, 22b) adapted to engage intimately with the medullary cavity and having the said surface texture suitable for connection to spongey bone tissue.

2. A component according to claim 1, characterised in that the core (15) has a dorsal strip (14) in the frontal plane which is on the convex side of the curvature and which is adapted to engage in the trochanter (3).

3. A component according to claim 1 or 2, characterised in that the core (15) is formed at its bottom end (16) with an elongate slot (l6a) In the frontal plane.

4. A component according to any of claims 1–3, characterised in that the shim bearing surfaces (21a, 22a) have a layer (23, 24) of a biologically compatible polymer.

5. A component according to any of claims 1–4, characterised in that the frontal surfaces (17, 18) of the core and the bearing surfaces (21a, 22a) of the shims have complementary guide means (17a, l8a; 25, 26) adapted to guide the shims (21, 22) relatively to the core (15) in the medullary cavity.

6. A component according to claim 5, characterised in that the guide means take the form in the case of the core (15) of a dovetail groove (l7a, 18a) in each frontal surface (17, 18) and in the case of each shim (21, 22) of a conical-head stud or the like which projects from the bearing surface.

7. A component according to claim 6, characterised In that the conical-head stud (25, 26) is disposed towards the bottom part of the shim (21, 22).

8. A component according to claims 6 or 7, characterised in that the conical-head stud (25, 26) is made of a biologically compatible polymer with a screw screwed into the bearing surface of the shim (21, 22).

9. A component according to any of claims 1–8, characterised in that each shim (21, 22) is formed with a cavity which is open to the top part of the shim and closed by a perforated wall along the convex surface (21b, 22b).

10. A component according to claim 7, characterised in that, when the bases of the grooves (17a, 18a) In the anterior and posterior surfaces (17, 18 respectively) of the core (15) are strictly parallel to the frontal plane, the conical studs (25, 26) projecting from the shims (21, 22) bear by way of a terminal portion on the bases of the corresponding grooves (17a, 18a) so as to limit displacement of the core (15) relatively to the shims (21, 22) purely to a translational movement in the frontal plane, whereas in their bottom part the surfaces (17, 18) merge into the general narrowing of the core.

EP 0 196 258 B1

1/1.

FIG.1

FIG. 2

FIG.3

FIG.5A

FIG. 4

FIG.5B